# EUROPEAN PATENT SPECIFICATION

(11) **EP 4 146 352 B1**
(45) Date of publication and mention of the grant of the patent: **12.11.2025**
(21) Application number: 21722485.6
(22) Date of filing: 04.05.2021
(51) Int. Cl.: A62B 7/14, A61M 16/12, A61M 16/00, A61M 16/06

(54) **REBREATHER APPARATUS**
KREISLAUFATEMVORRICHTUNG
APPAREIL À CIRCUIT FERMÉ

(30) Priority: 07.05.2020 EP 20173566
(43) Date of publication of application: 15.03.2023
(60) Divisional application: 23169301.1
(73) Proprietor: Aviation Works Limited, London E2 8DD (GB)
(72) Inventor: WAKEFORD, Tim, London E2 8DD (GB); BOYLE, Rob, London E2 8DD (GB)
(74) Representative: Gill Jennings & Every LLP
(86) International application number: PCT/EP2021/061672
(87) International publication number: WO 2021/224226

(56) References cited:
- WO-A1-2020/016422
- US-A1- 2010 139 658

## Description

### Field of Invention

The present invention relates to an apparatus and method for providing a ventilation gas mixture to users. The invention further relates to a ventilation mask for ventilation or respiratory support in such an apparatus.

### Background

Each human body has its own typical oxygen saturation value, which varies according to age, situation (e.g. altitude), and clinical condition. This value indicates the proportion of oxygen-charged haemoglobin in the blood, which in turn gives an indication of the efficiency with which breathing and oxygen transport is carried out in the body. Oxygen under-saturation is caused either by an oxygen partial pressure in the environment which is too low for humans (for example at an altitude above 10,000 ft. or 3048 m) and/or because of health disorders. The effects of oxygen under-saturation can be reduced by ventilating a person with oxygen.

In terms of ventilation, the concentration of oxygen in typically supplied ventilation air can be adjusted between a normal concentration of 21 % up to 100% of the gas mixture according to needs. During ventilation, pure oxygen flows through a supply tube into the mask and finally reaches the nose and mouth when the person using the mask breathes in. In some cases, pure oxygen is fed directly into the lungs using an intubation system. The oxygen supply is typically stored in pressure cylinders and connected to the mask via a series of supply tubes. The oxygen supply cylinders are often large and bulky, making them difficult to transport, in the case of hospital patients or activities requiring portable ventilation such as mountaineering or skydiving. Additionally, due to the size of the cylinders they often take up a lot of space, which is problematic in circumstances in which space is at a premium, for example aircraft ventilation.

In particular, in relation to aviation, due to the limited space and weight capacity of aircraft, the oxygen reserve can be increased only at the cost of cargo or the maximum number of passengers. A system that provides improved ventilation of the aircraft passengers and crew in case of cabin depressurization would result in less oxygen having to be carried aboard the aircraft, thus reducing the overall weight of the aircraft. Carrying a smaller quantity of oxygen also reduces the risk of fire which is associated with the fire accelerant effect of oxygen.

Further in relation to aviation, the duration of emergency oxygen supply in an aircraft is limited by the size of the emergency oxygen system, which is typically either a chemical or gaseous oxygen storage system. Since the size of the emergency oxygen system is primarily governed by size and weight restrictions of the aircraft, as previously mentioned, this effectively means that the size of the emergency oxygen system has a direct impact on the efficiency and fuel requirements of the aircraft. Further, since existing emergency oxygen systems are duration limited, the routes that aircraft can fly are often constrained by the performance of the emergency oxygen system (e.g. flight over large expanses of high terrain is not permitted). This extends route lengths and durations, and contributes to higher environmental impacts. WO2020/016422 describes an apparatus for preparing a ventilation gas mixture. It would be advantageous to provide an improved ventilation system for users which addresses at least some of the above-identified problems.

### Summary of Invention

The invention is defined by the subject-matter of the independent claims 1 and 15.

It has been found that a gas mixture to be used for ventilation which includes a carbon dioxide component allows a user to more efficiently use oxygen. Since the amount of oxygen required by a user varies depending on the altitude of the user, the corresponding additional carbon dioxide component varies according to altitude.

Advantageously, rather than supplying the carbon dioxide into a respiration mask from a long duration storage system (e.g. a carbon dioxide chemical generator or a pressurised carbon dioxide canister), the apparatus and systems described herein captures and uses the carbon dioxide from the user's own exhaled breath. This results in a system that is much simpler and less cumbersome, due to eliminating the carbon dioxide storage system. Furthermore, avoiding the need for a pressurised carbon dioxide canister provides a safer system with a reduced size and weight.

The altitude sensor may comprise a pressure sensor. This provides a simple means of determining the altitude at which the system is operating. In some examples, the pressure sensor may be arranged to determine the pressure of the ambient air and further configured to send the ambient air pressure to the control system. In this case, the altitude at which the system is operating at is determined based on the pressure of the ambient air surrounding the system.

In other cases, the pressure sensor may be arranged to determine the pressure of the exhaled air and further configured to send the exhaled air pressure to the control system. In this case, the altitude at which the system is operating at is determined based on information relating to a user's breathing. In this case, the pressure sensor may be located within the gas mixing device. This ensures that the pressure sensor is located in close proximity to the user's mouth, providing a more accurate determination of the pressure of the user's exhaled breath.

In other developments, the pressure sensor may be located within the first gas feed. In this case, the pressure sensor may determine the pressure of the user's exhaled air as it enters the first gas feed during a user's exhalation phase.

In some examples, the apparatus may further comprise an oxygen sensor. The oxygen sensor may be configured to determine the partial pressure of oxygen within the gas reservoir and further configured to send the partial pressure of oxygen to the control system. The control system may subsequently be configured to control the flow of oxygen through the first gas feed based on the partial pressure of oxygen in the gas reservoir.

The apparatus may also comprise a carbon dioxide sensor. The carbon dioxide sensor may be configured to determine the partial pressure of carbon dioxide within the gas reservoir and further configured to send the partial pressure of carbon dioxide to the control system. The control system may subsequently be configured to control the flow of oxygen through the first gas feed based on the partial pressure of carbon dioxide in the gas reservoir.

It should be noted that the apparatus may include, in a first alternative configuration, both an oxygen sensor and a carbon dioxide sensor. In a second alternative configuration the apparatus may include either an oxygen sensor and a carbon dioxide sensor.

The use of at least one additional sensor, namely an oxygen sensor and/or a carbon dioxide sensor allows the control system to make further adjustments to the amount of oxygen being supplied to the first gas feed based on information which is specific to a particular user. This allows the system to adjust standardised data available in look-up tables, databases, or algorithms to the particular needs and requirements of the user in question.

In some arrangements, the gas mixing device comprises a third gas feed configured to receive ambient air. In this case, the control system may be configured to control the flow of ambient air through the third gas feed into the gas mixing device based on the received altitude information. This ensures that only the amount of ambient air required at any given altitude is able to enter the system, ensuring that the ventilation gas mixture being prepared is optimal for that given altitude.

In some arrangements, the control system may be configured to control the flow of ambient air through the third gas feed into the gas mixing device based on the partial pressure of carbon dioxide. The control system may be configured to control the flow of ambient air through the third gas feed into the gas mixing device based on the partial pressure of oxygen. Thus, the control system may be configured to control the flow of ambient air through the third gas feed into the gas mixing device based on at least one of received altitude information, carbon dioxide partial pressure, oxygen partial pressure or any combination of these variables.

In some examples, the control system may be configured to control the supply of oxygen through the first gas feed such that oxygen is supplied to the gas mixing device as a at least one pulse. In this case, each breathing cycle may comprise a single pulse of oxygen. Alternatively, the control system may be configured to control the supply of oxygen through the first gas feed such that oxygen is supplied to the gas mixing device as a plurality of pulses. In this case, each breathing cycle may comprise two or more pulses of oxygen. Each of the at least one pulses or the plurality of pulses may comprise a predetermined volume of oxygen. This may ensure that the system is only supplying oxygen to the gas mixing device when it is required, resulting in a more efficient supply system.

In some examples, each pulse in the plurality of pulses comprises the same volume of oxygen. However, in other examples, at least two pulses in the plurality of pulses comprise a different volume of oxygen. Thus, the control system is configured to adjust the amount of oxygen being supplied to the first gas feed in real time, resulting in a more efficient system because only the amount of oxygen that is required at any given time is supplied to the first gas feed.

Alternatively, the control system may be configured to control the supply of oxygen through the first gas feed such that oxygen is supplied to the gas mixing device as a continuous flow of oxygen. In this case the user may always have a supply of oxygen available to them during their breathing cycle.

The control system may be any component which is able to adjust the flow of oxygen through the first gas feed. In some examples, the control system is an electrical component (for example a processor as part of a computer system) which is able to receive data and/or signals from an altitude sensor and send a corresponding signal the first gas feed to control the flow of oxygen. In other examples, the control system is a mechanical component (for example a barometric pressure sensor) which is able to control the flow of oxygen through the first gas feed based on mechanical movement of the control system.

Thus, throughout the description, control system refers to any component, either mechanical or electrical, which controls the flow of oxygen through the first gas feed based on a response, either mechanical or electrical, of an altitude sensor. Thus, in some cases, the control system comprises a plurality of components, for example, an electrical control system comprises a memory and a processor. In other cases the control system comprises a single component, for example a mechanic control system may only include a barometric sensor.

According to the invention, the gas mixing device is a ventilation mask. The ventilation mask is arranged to be worn by a user. In this case, no extra equipment is required to supply the ventilation gas mixture to a user. Further, this arrangement ensures that the prepared ventilation gas mixture is supplied quickly and efficiently to the user, without being fed through multiple system components.

Preferably, the pressure sensor is configured to determine pressure information by detecting a breathing characteristic of a user. The breathing characteristic may be at least part of a breathing cycle, for example an inhalation phase and/or an exhalation phase. More preferably, the pressure sensor is configured to determine the pressure of the exhaled air from a person.

In a further development there may be provided the use of the apparatus as described above, a ventilation mask as described above, and/or a method as described above for ventilating a person in an aircraft. This relates both to use for the pilots, the cabin crew and for the aircraft passengers.

In a further development there may be provided the use of the apparatus, the ventilation mask, and/or the method for ventilating a person during high altitude activities. In this context, high altitude activities include, but are not limited to mountaineering, skydiving, and parachuting. The apparatus, the ventilation mask, and/or the method may be used for military applications for example by military personnel during aerial delivery (including but not limited to air load masters and military parachutists).

In a further development there may be provided the use of the apparatus as described above, a ventilation mask as described above, and/or a method as described above for ventilating a person during medical applications. In this context medical applications include, but are not limited to, a person in a medical environment such as a hospital undergoing a form of medical treatment such as surgery.

The apparatus, systems, and methods described herein, as well as a ventilation mask usable in the apparatus, systems, and methods as described, therefore enable a ventilation gas mixture of variable composition to be prepared simply and provides for advantageous uses.

As will be appreciated any numerical values specified throughout are not exact and can include small variations (such as but not limited to standard mathematical rounding conventions) either side of the numerical value which bring about the same technical effect as the specified value.

As will be appreciated all of the above aspects and developments may be combined together in any combination and they may be used together in combination with each other.

### Brief Description of Drawings

Features and practical uses of the invention will be described, by way of example only, with reference to the accompanying drawings in which:
Figure 1 is a schematic illustration of a example ventilation apparatus;
Figure 2 is a schematic illustration of another example ventilation apparatus;
Figure 3 is a schematic illustration of a third example ventilation apparatus.
Figure 4 is a schematic illustration of an inhalation phase of a breathing cycle using an example ventilation apparatus;
Figure 4a is a schematic illustration of an inhalation phase of a breathing cycle using another example ventilation apparatus;
Figure 5 is a schematic illustration of an exhalation phase of a breathing cycle using an example ventilation apparatus; and
Figure 5a is a schematic illustration of an exhalation phase of a breathing cycle using another example ventilation apparatus.

### Specific Description

It has been found that relatively small quantities of carbon dioxide added to ventilation air allow the human body to better absorb and utilise oxygen supplied during ventilation. This can be achieved by recycling the exhaled air from a user, containing carbon dioxide, back into the ventilation apparatus so that it can be mixed with the supplied oxygen to create an optimal ventilation gas mixture.

The following description will explain how the exhaled carbon dioxide from the user's breath can be re-used and mixed with oxygen to form a carbon dioxide enriched ventilation gas mixture.

In general, the captured carbon dioxide from the exhaled breath of a user is recycled back into the ventilation system at quantities between 0% and 16% carbon dioxide by volume to form the ventilation gas mixture. The amount of carbon dioxide which is recycled back into the ventilation gas mixture depends on a number of factors, including the altitude at which the ventilation gas is required to be used, the partial pressure of oxygen, system performance requirements (for example, manufacturing limits of the system components), and physiological requirements of the human body. The amount of carbon dioxide that is needed and for what duration can be determined in order to first improve oxygen saturation and then maintain a satisfactory level. The ventilation gas mixture is then passed to a ventilation mask to be inhaled by the user.

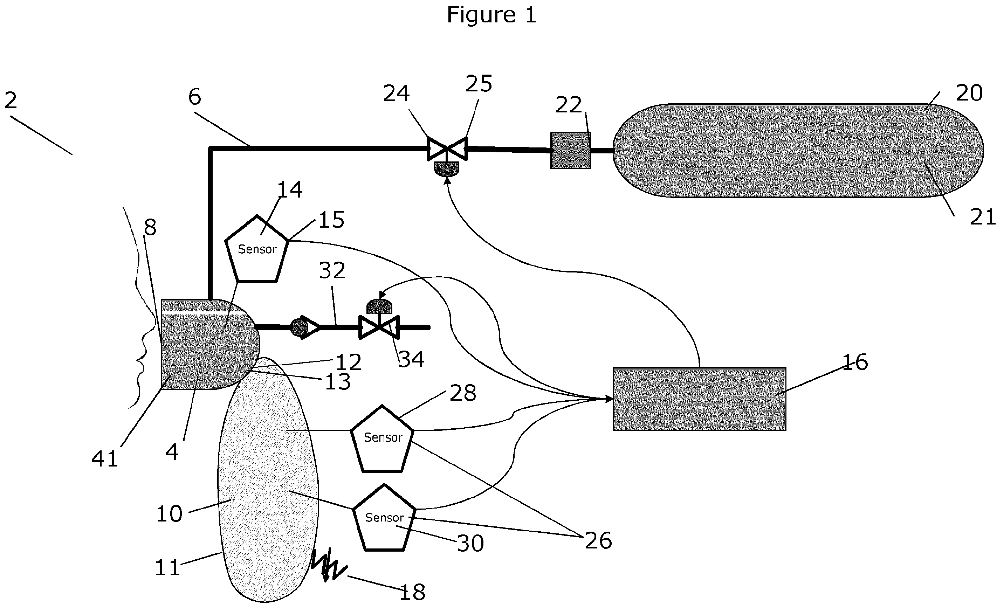

Figure 1 shows an example system 2 for preparing a ventilation gas mixture. The system 2 comprises a gas mixing device 4, which receives oxygen through a first gas feed 6 and receives exhaled air from a person through an air inlet 8. A gas reservoir 10 is in fluid communication with the gas mixing device 4 such that the exhaled air can flow into the gas reservoir 10 from the gas mixing device 4 through a second gas feed 12. The system further comprises an altitude sensor 14, which is arranged to determine the altitude of the system 2. A control system 16 is connected to the altitude sensor 14 so that it can receive and process the altitude information. The control system is connected to a power source (not shown), for example a battery or mains power supply, Based on the received altitude, the control system 16 controls the flow of oxygen through the first gas feed 6 into the gas mixing device 4 where it is combined with exhaled air that has been re-supplied from the gas reservoir 10 through the second gas feed 12 in order to prepare the ventilation gas mixture.

As can be seen in Figure 1, the gas mixing device 4 is in the form of a ventilation mask 41 which can be worn by a user. The gas reservoir 10 is in the form of a re-breathing bag 11. In this example, the gas reservoir 10 is shown as being coupled directly to the gas mixing device 4 with the second gas feed 12 taking the form of an aperture or through-hole 13 between an external wall of the gas mixing device 4 and an external wall of the gas reservoir 10. However, in other examples, the gas reservoir 10 may be indirectly coupled to the gas mixing device 4 with the second gas feed 12 taking the form of a gas pipe or tube between an outlet in an external wall of the gas mixing device 4 and an outlet in an external wall of the gas reservoir 10. Gases are able to freely flow through the second as feed 12, between the gas mixing device 4 and the gas reservoir 10.

The gas reservoir 10 is made from any suitable material that is impermeable to gases, for example polyvinylchloride (PVC) in order that the gas reservoir 10 is able to receive and store the exhaled air received by the user of the system 2. Since the air contained within the gas reservoir 10 cannot escape through the walls, or external surface, of the gas reservoir 10, a one-way valve 18, which may also be referred to as an exhaust valve, is provided within the wall of the gas reservoir 10. This allows air to escape from the gas reservoir 10 to the surroundings if the pressure inside the gas reservoir 10 becomes too great as a result of a user exhaling into the gas reservoir 10 for a prolonged period of time. In this case, the exhaust valve 18 is in closed when the pressure within the gas reservoir 10 is below a certain threshold, so that the exhaled air is stored within the gas reservoir 10 and prevented from being exhausted to the surroundings. Once the pressure inside the gas reservoir 10 exceeds this threshold, the exhaust valve 18 opens to allow excess air to escape from the gas reservoir 10, preventing the gas reservoir 10 from exploding due to high pressures.

As mentioned, oxygen is supplied to the gas mixing device 4 through the first gas feed 6, which is connected to an oxygen source 20. As shown in Figure 1, the oxygen source 20 takes the form of a pressurised cylinder 21 comprising nominally 100% oxygen. The first gas feed 6 comprises a pressure regulator 22 in order to control the pressure of the oxygen that enters the system 2 via the first gas feed 6 from the oxygen source 20. This ensures that the oxygen being fed to the gas mixing device 4 is of a slightly higher pressure compared to the exhaled air also received by the gas mixing device 4, to allow flow throughout the breathing cycle. Although Figure 1 shows the pressure regulator 22 being positioned separate from the oxygen source 20, in some examples, the pressure regulator 22 may form part of the oxygen source 20.

In addition to the pressure regulator 22, the first gas feed 6 also comprises a flow regulator 24, which is arranged to control the flow of oxygen from the oxygen source 20 through the first gas feed 6 and into the gas mixing device 4. The flow regulator 24 is able to maintain a pre-defined flow rate, which is determined by and controlled by the control system 16. As shown in Figure 1, the flow regulator 24 takes the form of a valve 25 which is in communication with the control system 16. Although shown as separate from the oxygen source 20, in some cases the flow regulator 24 may form part of the oxygen source 20, for example a valve on a pressurised oxygen cylinder. Alternatively, the flow regulator 24 may form part of the control system 16 as a component installed on a circuit board of the control system.

The control system 16 opens and closes the flow regulator 24 to adjust the amount of oxygen that is allowed to flow into the system 2, based on the altitude of the system 2. Different operating altitudes require different amounts of oxygen to be supplied to the gas mixing device 4 in order to maintain the partial pressure of oxygen within the ventilation gas mixture that will be breathed by the user. A database, or look-up table or algorithms, of required oxygen partial pressures at different altitudes can be accessed by the control system such that the correct amount of oxygen can be supplied to the system 2.

The algorithm is therefore used to inform the decision making of the control system 16, using data that has been acquired through testing, based on pre-determined system performances and typical performances of the human body and its ability to use oxygen at various different altitudes and operating conditions.

The altitude sensor 14, which is arranged to determine the altitude of the system 2, takes the form of a pressure sensor 15. In the example shown in Figure 1, the pressure sensor 15 is in communication with the gas mixing device 4 and arranged to determine the pressure of the air, in particular the received exhaled air, in the gas mixing device. Although the altitude sensor 14 is illustrated as being separate from the gas mixing device 4, in some cases the altitude sensor may be located within the gas mixing device 4. For example, if the gas mixing device 4 is a ventilation mask, the altitude sensor 14 may be a pressure sensor located within the mask and arranged to determine the pressure of the user's exhaled breath. In other cases, the altitude sensor 14 may form part of the control system 16, being housed within a circuit board of the control system 16.

The altitude sensor 14 can be thought of as being arranged to determine the altitude of the system based on a breathing characteristic of the user of the system. As has been described in relation to the example shown in in Figure 1, the altitude sensor 14 determines the pressure of the user's exhaled air. In this case, the breathing characteristic is the breathing cycle of the user. That is to say, the altitude sensor 14 is arranged to detect the inhalation and exhalation of the user, based on changes in pressure in the gas mixing device 4.

The ability to detect the breathing cycle of the user, i.e. detect when the user is inhaling and exhaling, enables the control system 16 to supply oxygen to the gas mixing device more efficiently. This is because the flow of oxygen into the first gas feed 6 can be controlled such that oxygen flows into the first gas feed 6 while a user inhales. Whilst the system would still function if oxygen were allowed to flow into the first gas feed 6 during an exhalation, this arrangement may result in the user causing some of the supplied oxygen to flow back along the first gas feed 6, away from the gas mixing device 4 and back towards the oxygen supply source 20. In this case, the required oxygen would remain in the first gas feed 6 until the user inhales.

The altitude sensor 14 which is in communication with the control system 16 sends the determined altitude information to the control system 16, which can in turn determine the correct amount of oxygen to supply to the system 2, based on the data within the algorithm. The flow of oxygen through the first gas feed 6 is therefore controlled as a function of altitude.

The control system 16 can be arranged to supply oxygen into the system 2 in a number of different ways. In a first case, the control system 16 may be arranged to control the supply of oxygen from the oxygen source 20 such that the flow of oxygen into the first gas feed 6 is continuous. In this case, the control system 16 adjusts the flow regulator 24 so that it is always in at least a partially open position, providing a continuous supply of oxygen to the gas mixing device 4. If a greater supply of oxygen is needed, for example due to higher altitudes, then the control system 16 adjusts the flow regulator 24 to allow a greater volume of oxygen to flow through the first gas feed 6. If only a small supply of oxygen is required, for example due to lower altitudes, the control systems 16 adjusts the flow regulator 24 to allow a lower volume of oxygen to flow through the first gas feed 6.

In a second case, the control system 16 may be arranged to control the supply of oxygen from the oxygen source 20 such that the flow of oxygen into the first gas feed 6 is pulsed. In this case, the control system 16 adjusts the flow regulator 24 so that it alternates between an open and a closed position, providing a supply of oxygen to the gas mixing device 4 at discrete intervals. The intervals between each pulse of oxygen could be the same, i.e. the length of time of each interval is constant, or at least some of the intervals, in some cases all of the intervals, could be different i.e. the length of time of at least two intervals is different.

For optimal efficiency, the flow of oxygen into the first gas feed 6 would be pulsed such that oxygen is only supplied to a user when it is required. However, a non-pulsed i.e. continuous oxygen supply system would still provide performance improvements over existing systems.

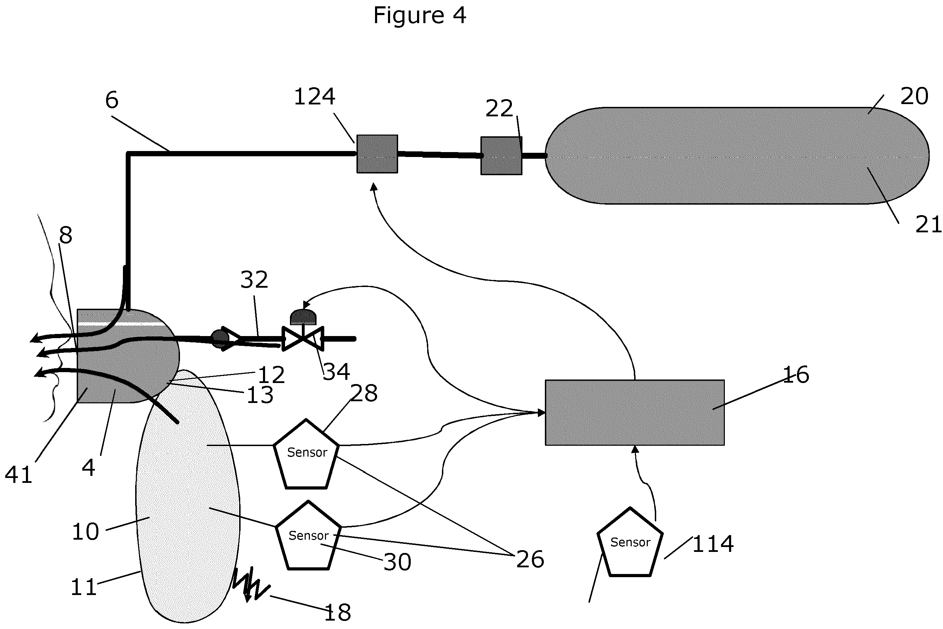

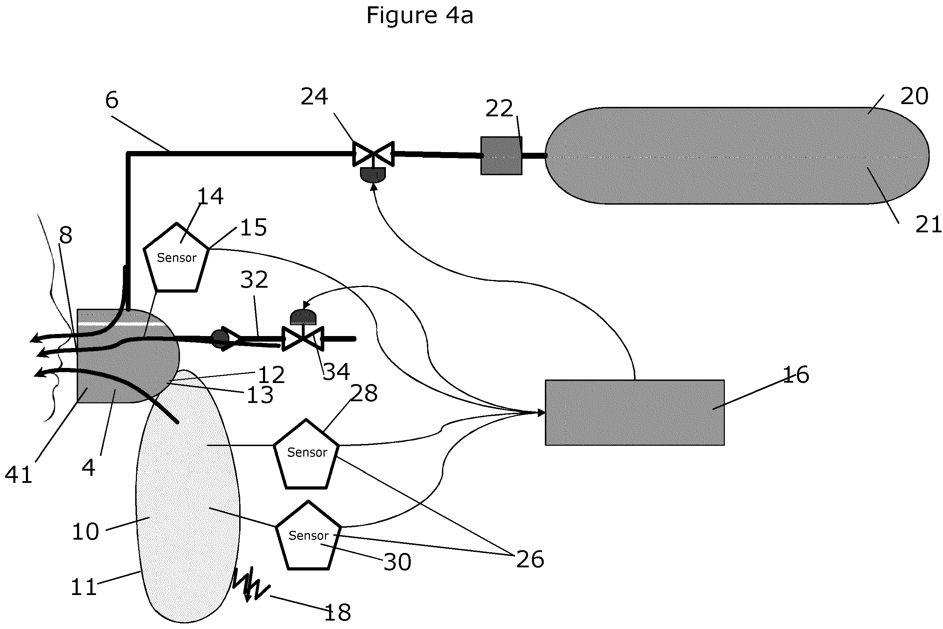

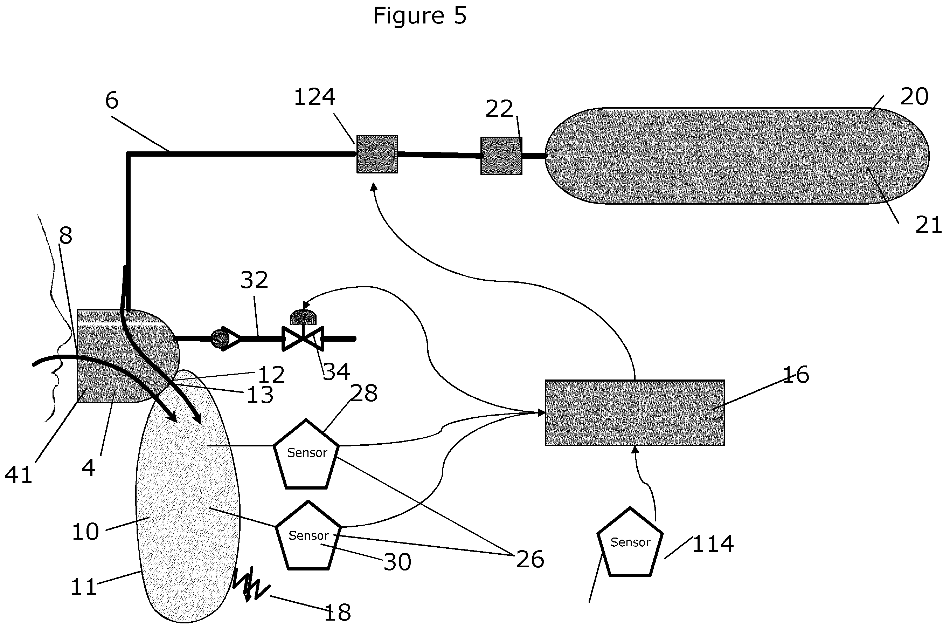

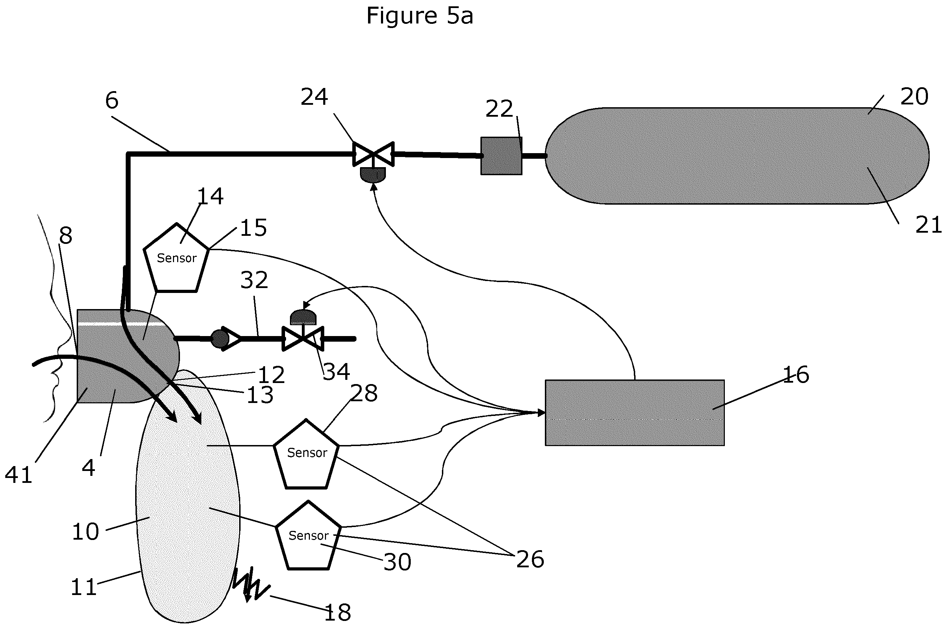

During usage, when the user first inhales, oxygen from the oxygen source 20 is inhaled straight into the user's lungs, as shown in Figure 4. When the user subsequently exhales, any oxygen flowing from the first gas feed 6, as well as the user's exhaled breath is fed into the gas reservoir 10, as shown in Figure 5. During subsequent inhalation, the user will inhale air from both the gas reservoir 10 and the first gas feed 6 which has been combined in the gas mixing device 4 to form the ventilation gas mixture.

The above-described system 2 can be used in a number of different fields, in applications that require a user to be supplied with a ventilation gas mixture. Some of the applications include aviation, mountaineering, skydiving, and medical applications for example in hospitals.

Whilst a general system has been described above, a number of modifications can be made to the system, as will be described in the following.

As will be appreciated, the oxygen requirements of a user will vary between users, depending on a number of factors including age, gender, underlying medical conditions, etc. Algorithms of oxygen requirements at different altitudes typically comprise data representative of a large proportion of the general population, for example a middle-aged male.

In order to more accurately determine the oxygen requirements of the user (for example, an infant may require less oxygen than an adult) the system may include additional sensors 26 arranged to provide additional information about the system 2 to the control system 16. In particular, the additional sensors 26 may comprise an oxygen sensor 28 and a carbon dioxide sensor 30, as shown in Figure 1. The oxygen sensor 28 and carbon dioxide sensor 30 are arranged to detect the partial pressures of oxygen and carbon dioxide respectively within the system 2 and feed this information back to the control system 16. As illustrated in Figure 1, the oxygen sensor 28 and carbon dioxide sensor 30 are coupled to the gas reservoir 10 and are arranged to detect the partial pressures of oxygen and carbon dioxide within the gas reservoir 10. Although these additional sensors 26 have been shown as external to and separate from the gas reservoir 10, in some cases, the additional sensors 26 may be located within the gas reservoir 10.

The oxygen and carbon dioxide sensors 28, 30 will measure the relative amount of oxygen and carbon dioxide present in the user's exhaled breathe. This information is then be sent to the control system 16 in order to calculate the optimal amount of oxygen to supply to the first gas feed 4. That is, the control system 16 will look up the recommended amount of oxygen to supply to the user using the altitude information and the database. This recommended amount is then adjusted, for example increased or decreased, based on the information received from the additional sensors 26. The use of additional sensors 26 to adjust the supply of oxygen into the system 2 therefore ensures that each user is provided with an optimal amount of oxygen based on their needs. Furthermore, the control system 16 is able to adjust in real time the supply of oxygen to the user, based on the user's needs as they change over time. Thus, the additional sensors 26 provide a more efficient system 2 overall.

As mentioned previously, the altitude sensor 14 detects a breathing characteristic of the user, which is used to determine the altitude. Similarly, any additional sensor 26 present in the system 2, including the oxygen sensor 28 and/or carbon dioxide sensor 30, may also be thought of as detecting a breathing characteristic of the user. In particular, the oxygen and carbon dioxide levels of the user's exhaled air in the gas reservoir 10 can be monitored using corresponding oxygen and carbon dioxide sensors. Fluctuation in one or more of the quantities of these gases would indicate the user's breathing cycle. For example, an increase in carbon dioxide would indicate that a user is exhaling.

Whilst the system 2 in Figure 1 comprises both an oxygen sensor 28 and a carbon dioxide sensor 30, in some cases only one additional sensor (for example only a carbon dioxide sensor 30 or only an oxygen sensor 28) could be provided. In this case, either the partial pressure of oxygen or the partial pressure of carbon dioxide would be sent to the control system 16 and used to modify the recommended amount of oxygen for the user. It should also be noted that in some cases, such as the general system described previously, no additional sensors 26 are included. In this case, the recommended amount of oxygen to be supplied to a user at a particular altitude is not adjusted.

In summary, the control system 16 calculates how much oxygen to supply to the gas mixing device 4 based upon a combination of the following parameters: ambient pressure determined using the altitude sensor 14, partial pressure of oxygen in the gas reservoir 10 determined using an oxygen sensor 28 (where present), partial pressure of carbon dioxide in the gas reservoir 10 determined using a carbon dioxide sensor 30 (where present), and pre-defined lookup tables.

In other developments of the system 2, there is provided a third gas feed 32 arranged to supply ambient air to the gas mixing device 4 as shown in Figure 1. Once the ambient air has entered the system 2, it can be combined with the oxygen from the first gas feed 6 and the exhaled air from the second gas feed 12 to form the ventilation gas mixture.

In general, the ambient air will comprise a mixture of oxygen, carbon dioxide, and nitrogen. By allowing the ambient air to enter the gas mixing device and combine with the oxygen and exhaled air from the user, the relative proportions of the gas making up the ventilation gas mixture can be adjusted.

The ambient air received via a third gas feed 32 is therefore used to control the proportion of carbon dioxide present within the ventilation gas, and ensure that the amount of carbon dioxide remains at the required level.

Control of the ambient air into the gas mixing device 4 is provided by the control system 16 which is coupled to the third gas feed 16. In particular, the control system 16 is in communication with a valve 34 within the third gas feed 16 and arranged to adjust the position of the valve between open and closed positions, depending on the amount of ambient air to be allowed to enter the system 2.

Specifically, the control system 16 opens and closes the valve 34 in the third gas feed 32 based on information received from the altitude sensor 14, the algorithm of pre-determined data, and any additional sensors 26 if they are present. By combining all this received data, the control system is able to make further necessary adjustments to the ventilation gas mixture by allowing ambient air to enter the system 2. The valve 34 may also be referred to as an ambient air dilution valve, and takes the form of a one way valve which allows ambient air into the system 2 but not out of the system 2.

Since the control of the valve 34 by the control system 16 is based on altitude information received from the altitude sensor 14, control of the valve 34, i.e. control the proportion of ambient air into the third gas feed 32, can be thought of as being based on the breathing characteristic of the user, which is detected using the altitude sensor 14.

In other words, sensing of the breathing characteristic is used to determine how much ambient air to allow into the gas mixing device 4 in order to control the proportion of carbon dioxide present with the prepared ventilation gas mixture such that it remains at the required level.

As mentioned, the system 2 can be used for a number of different applications which require ventilation. In some applications, such as medical applications including ventilation equipment in hospitals, the third gas feed is optional. In other applications, such as aviation applications including emergency oxygen masks for passengers and crew members, the third gas feed is generally present.

The main function of the third gas feed 32 is to act as a dilution gas feed, which allows dilution of carbon dioxide within the ventilation gas mixture whilst allowing a reduction in oxygen supplied from the first gas feed 6. This is because ambient air comprises oxygen and nitrogen, with negligible amounts of carbon dioxide. When the altitude of the systems decreases, for example when an aircraft descends, the partial pressure of oxygen increases and so less supplemental oxygen is required to be supplied from the first gas feed 6 to the gas mixing device 4 in order to prepare a suitable ventilation gas mixture. However, as the flow of oxygen through the first gas feed 6 is reduced, the amount of carbon dioxide present in the gas reservoir 10 increase. Therefore, by opening the third gas feed 32 the carbon dioxide present in the gas reservoir 10 can be diluted to the correct levels for the ventilation gas mixture.

In practice, since opening of the third gas feed 32 will also dilute the partial pressure of oxygen in the gas mixing device 4, a slight increase in oxygen being supplied from the first gas feed 6 is required. It should be noted however, that this slight increase is not as large as would be required to dilute the carbon dioxide levels if the third gas feed 32 were not present at all. The presence of the third gas feed 32 is therefore a method of increasing system efficiency as the oxygen source 20 will last longer.

The third gas feed 32 can therefore be used to adjust the amount of oxygen present in the ventilation gas mixture, without allowing levels of carbon dioxide to build up to unsafe levels. In particular, when less oxygen is required, due to the reduction in altitude, the amount of oxygen supplied by the first gas feed 6 is reduced. However, there is a risk that the amount of carbon dioxide stored in the gas reservoir 10 may build up over time, and so the third gas feed 32 can help dilute the carbon dioxide levels in the ventilation gas mixture.

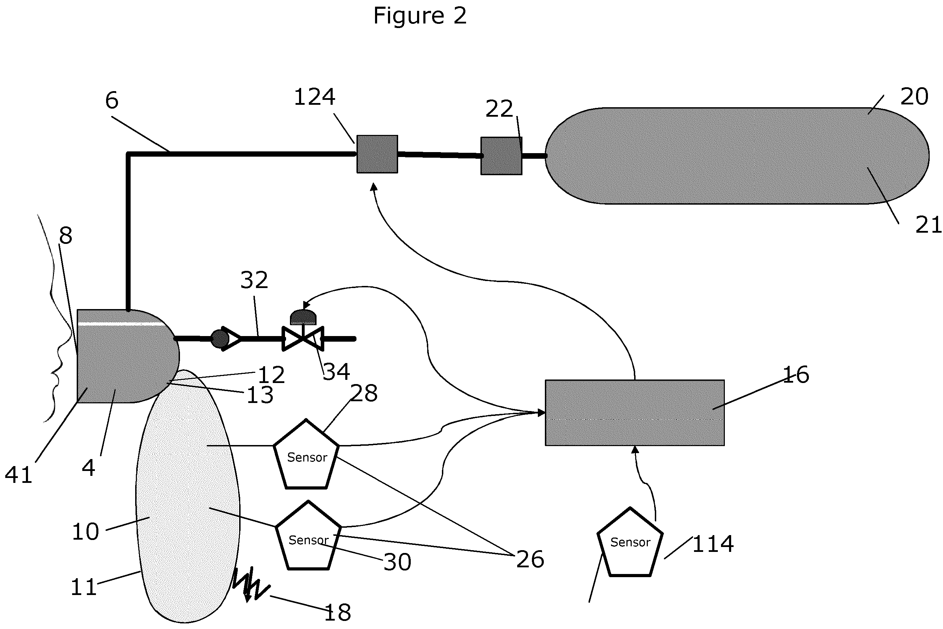

Figure 2 illustrates an alternative system for use in preparing a ventilation gas mixture. The same reference numerals have been used to identify the same features that have been described previously.

In this example, the altitude sensor takes the form of an air pressure sensor 114, arranged to determine the air pressure of the ambient air. This air pressure sensor 114 is in communication with the control system 16 and provides the control system 16 with the measured ambient air pressure, which then uses this information to determine the altitude of the system. This determination is based on pre-defined algorithms or, lookup tables in a database, which includes data corresponding to altitudes at various air pressures.

As can be seen in Figure 2, the flow restrictor 124 takes the form of a pressure controlled flow regulator, specifically a barometrically controlled flow regulator 124. The control system 16 is in communication with the flow regulator 124 and adjusts the flow regulator 124 based on the altitude calculated using the ambient air pressure received from the air pressure sensor 114. As before, different altitudes correspond to different recommended amounts of oxygen to be supplied to the system and so the control system 16 is able to control the flow of oxygen into the first gas feed 6 based on the determined altitude.

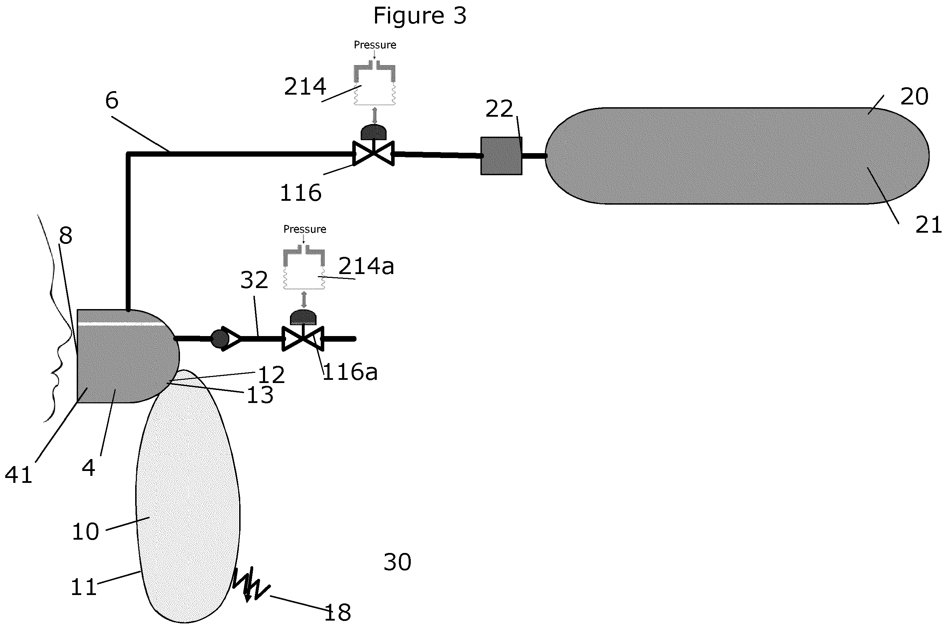

Figure 3 illustrates another alternative system for use in preparing a ventilation gas mixture. The same reference numerals have been used to identify the same features that have been described previously.

In this example, the altitude sensor takes the form of a pressure sensor, specifically a barometric sensor 214. The barometric sensor is arranged to determine the pressure of the ambient air. Additionally, the control system takes the form of a control valve 116, which is able to control the flow of oxygen from the oxygen source 20 and into the first gas feed 6. The control valve 116 can be moved between an open position and a closed position, as well as intermediate partially open positions, in order to control the amount of oxygen that is allowed to flow from the oxygen source 20 to the first gas feed 6.

The barometric sensor 214 is in communication with the control system 116 which adjusts the flow of oxygen through the firs gas feed 6 based on the altitude determined by the barometric sensor 214. When the barometric sensor 214 comprises a pressure-dependent component which moves or changes shape, depending on the pressure of the air around it. This pressure-dependent component is located substantially next to the control valve 116 such that any movement of the pressure-dependent component affects the control valve 116 and therefore the flow of oxygen through the first gas feed 6. In particular, the barometric sensor 214 will cause the control valve 116 to move between open and closed positions, as well as between partially open positions, based on the movement of the pressure-dependent component. As before, different altitudes correspond to different recommended amounts of oxygen to be supplied to the system and so the control system 116 is able to control the flow of oxygen into the first gas feed 6 based on the altitude.

Similarly, the third gas feed 32 may comprise a control system 116a and a barometric sensor 214a. Again, the control valve 116a is arranged to control the flow of ambient air into the third gas feed 32 based on movement of a pressure-dependent component within the barometric sensor 214a, as described above.

Figure 3 therefore illustrates a mechanical system compared to the electrical systems of Figures 1 and 2. In Figures 1 and 2, the flow of oxygen through the first gas feed 6 is controlled by the control system 16 which receives data and signals via an electrical connection from an altitude sensor 14. The control system 16 then sends a signal to the flow restrictor 24 via an electrical connection in order to control the flow of oxygen. In Figure 3, the flow of oxygen through the first gas feed 6 is controlled by the control valve 116 which receives a mechanical response from the barometric sensor 216. The control valve 116 then moves between open and closed positions (or vice versa) or between first and second partially open positions (the first and second partially open position being different from each other such that each partially open position corresponds to a different amount of oxygen able to flow into the first gas feed 6) in order to control the flow of oxygen.

Figures 4 and 5 illustrate the flow of gases through the system during inhalation and exhalation. The system in these Figures is arranged such that the control system supplies a continuous supply of oxygen into the first gas feed 6. However, a similar sequence of events would be applicable for a system arranged to supply a pulsed supply of oxygen. It should be noted that whilst these Figures have been shown to include a barometrically controlled flow regulator 124, an ambient air pressure sensor 114, and a third gas feed 32, the flow of gases through the system is the same using the flow restrictor 24 and altitude sensor 14, as shown in Figures 4a,and 5a as well as when the third gas feed 32 is not present. In addition, the flow of gases through the system is the same when the system is a mechanical system, as shown in Figure 3. It should also be noted that the flow of gases through the system is the same when the oxygen is supplied to the first gas feed 6 as a series of pulses or as a continuous supply.

Looking first at Figure 4, an inhalation phase of a breathing cycle is shown. Generally, when the user inhales, oxygen supplied from the first gas feed 6 flows straight into the user's lungs via the gas mixing device 4. The user will also inhale air stored within the gas reservoir 10. When the system is used for the first time and the user takes their first breath in, the gas reservoir 4 will comprise a nominal amount of ambient air. In some cases, when the system is used for the first time, the system may either need to provide an initial amount of extra oxygen in order to sufficiently fill the lungs, or ensure that the third gas feed 32 is open (when present) to allow ambient air into the third gas feed 32, in order to prevent the user from attempting to inhale on an empty gas reservoir 10. During subsequent inhalations, the gas reservoir 4 will have collected and stored previously exhaled air from the user, and so the user will generally be inhaling their stored exhaled air. This exhaled air will be combined in the gas mixing device 4, just before it enters the user's mouth and lungs, to form the ventilation gas mixture.

Since the exhaled air comprises increased levels of carbon dioxide, when the user re-breathes this exhaled air in combination with the supplied oxygen, they will be breathing in an air mixture comprising increased levels of carbon dioxide. As discussed, the adding carbon dioxide to the ventilation gas mixture enables the user to more efficiently use the oxygen being supplied through the first gas feed 6.

In arrangements which also comprise the third gas feed 32, when this gas feed is opened, the user will additionally inhale an amount of ambient air, in combination with the supplied oxygen and re-supplied exhaled air, which is then mixed with the supplied oxygen and re-supplied exhaled air in the gas mixing device 4 to produce the ventilation gas mixture.

Some arrangements include a ventilation mask which forces the mask wearer to inhale the ventilation gas mixture, made up of oxygen supplied from the oxygen source 20 in combination with either air from the gas reservoir 10 (which includes exhaled carbon dioxide nominally up to 20% by volume), or a mix of ambient air and gas reservoir air. In the latter case, the ambient air is used in combination with oxygen supplied from the oxygen source, to dilute the carbon dioxide from the gas reservoir to required levels.

During inhalation, the flow restrictor 24 limits the flow of oxygen into the gas mixing device 4. The can be a limitation to a period of time (as in the pulsed arrangement), and/or flow rate

The limitation ensures that, during each respiratory cycle, only a preselected volume of oxygen is supplied which, in combination with the carbon dioxide, is sufficient to provide a suitable ventilation gas mixture for a given altitude.

The flow restrictor 24 is opened substantially immediately upon detection by the altitude sensor 14 of a negative pressure within the gas mixing device 4. The preselected volume of oxygen and carbon dioxide (and where applicable, nitrogen from ambient air) is delivered to the gas mixing device 4 in the correct quantities, for inhalation by the user, the quantities being controlled and determined by the control system 16 and algorithms or look-up tables, as explained previously.

In other words, the control system 16 is arranged to supply a volume of oxygen, carbon dioxide (and in some cases nitrogen), as a function of altitude, as determined based on air pressure.

In particular the preselected volume of oxygen comprises a volume of oxygen sufficient such that, when mixed with a preselected volume of carbon dioxide and oxygen from the gas reservoir 4 (and in some cases ambient air), the preselected volume of oxygen is sufficient to maintain a preselected oxygen saturation level at a determined altitude, noting that the presence of carbon dioxide in the ventilation gas mixture reduces the nominal amount of oxygen required to sustain the user.

Since the preselected volume of oxygen is delivered to the gas mixing device 4 during the inhalation phase, the flow restrictor 24 is opened for at least part of, in some cases all of, a user's inhalation phase.

Figure 5 illustrates the exhalation phase of the breathing cycle. Generally, when the user exhales, their exhaled breath will pass through the gas mixing device 4 and into the gas reservoir 10 via the second gas feed 12 where it is collected and stored. Any previously supplied, or continually flowing oxygen remaining in the gas mixing device 4 or the first gas feed 6 will also be forced into the gas reservoir 10 during exhalation.

The presence of the third gas feed 32 in arrangements which also include this feature does not affect the flow of gases during exhalation, since the third gas feed 32 comprises a one-way valve which does not allow air to exit the system via the third gas feed 32. During the exhalation phase, the flow restrictor 24 in the first gas feed 6 also remains closed.

Over time, the gas reservoir 4 therefore accumulates a volume of gas comprising oxygen, carbon dioxide, and nitrogen, either flowing from an oxygen source, from the ambient cabin air, or from air exhaled by the user.

As the skilled person will understand, there are a number of different configurations in which any of the above-described systems could be employed. Some of these configurations, and their corresponding applications, will be outlined below.

In a first application, the system may be used in combination with a ventilation mask for providing ventilation gas mixtures for use during aviation for aircraft crew or passengers. This system comprises a gas mixing device, a gas reservoir, first and second gas feeds, an altitude sensor, and a control system. The altitude sensor could be located in the gas mixing device, as in Figure 1, or could be an air pressure sensor, and in Figure 2. The system further comprises a third gas feed, as shown in Figure 2. Operation of this system, and the flow of gases through this system, is as described above.

In a first application, the system may be used in combination with a ventilation mask for providing ventilation gas mixtures for use during aviation for aircraft crew or passengers. This system comprises a gas mixing device, a gas reservoir, first and second gas feeds, an altitude sensor, and a control system. The altitude sensor could be located in the gas mixing device, as in Figure 1, or could be an air pressure sensor, as in Figure 2. The system further comprises a third gas feed, as shown in Figure 2. Optionally, the system may include a carbon dioxide sensor and/or an oxygen sensor as shown in Figure 1. Operation of this system, and the flow of gases through this system, is as described above. Preferably, the flow of oxygen through the system is controlled in a pulsed manner. However, a continuous supply of oxygen could also be used.

In a second application the system may be used in combination with a ventilation mask, a ventilation helmet, or intubation system for providing ventilation gas mixtures for use by medical patients or for use during medical procedures. This system comprises a gas mixing device, a gas reservoir, first and second gas feeds, an altitude sensor, and a control system. The altitude sensor could be located in the gas mixing device, as in Figure 1, or could be an air pressure sensor, as in Figure 2. Optionally, the system may include a carbon dioxide sensor and/or an oxygen sensor as showing in Figure 1. Optionally, the system further comprises a third gas feed, as shown in Figure 2. Operation of this system, and the flow of gases through this system, is as described above. Preferably, the flow of oxygen through the system is controlled in a pulsed manner. However, a continuous supply of oxygen could also be used. In some arrangements, the system may be coupled to a biological filter located in the second gas feed, in order to help prevent infectious pathogens being released into the environment surrounding the user.

With reference to the second application, an alternative system may be used in combination with a ventilation mask, a ventilation helmet, or intubation system for providing ventilation gas mixtures for use by medical patients or for use during medical procedures. This system is generally the same as the system described above in connection with the second application. However, in this application, the gas reservoir comprises an exhaust valve configured to provide an increased pressure restriction, facilitating operation of the system at pressures greater than ambient air pressure. By this we mean that the pressure within the gas mixing device is higher than the ambient pressure outside the system and so by configuring the exhaust valve to release a higher pressure, the system is forcing the ventilation gas mixture out of the gas mixing device at into a user's lungs under pressure. In this case, oxygen being supplied through the first gas feed is supplied to the gas mixing device at pressures that are greater than those of oxygen being supplied through the first gas feed when the system is being used for other applications. This has the advantage that the partial pressure of oxygen and carbon dioxide can be increased in order to make them more effectively used by the user, whilst still conserving the gases within the system which optimises gas usage. In some applications, this pressure restriction may be controlled based upon the altitude of the system, and/or the gas composition measured within the gas mixing device.

In a third application the system may be used in combination with a ventilation mask for providing ventilation gas mixtures for use during high altitude sports or activities, such as mountaineering, skydiving, or parachuting. This system comprises a gas mixing device, a gas reservoir, first and second gas feeds, an altitude sensor, and a control system. In this case, the gas mixing device takes the form of the ventilation mask, and is worn by the user. The altitude sensor may be located in the gas mixing device, as in Figure 1. In particular, in arrangements in which the control system is arranged to provide a pulsed supply of oxygen to the first gas feed, the altitude sensor is located within the ventilation mask. However, in arrangements in which the control system is arranged to provide a continuous supply of oxygen to the first gas feed, the altitude sensors could be located either within the ventilation mask, or external to the ventilation mask for example within the first gas feed. The system further comprises an oxygen supply, in the form of a compressed oxygen cylinder, configured to supply oxygen to the first gas feed. Optionally, the system may include a carbon dioxide sensor and/or an oxygen sensor as showing in Figure 1. Optionally, the system further comprises a third gas feed, as shown in Figure 2. Operation of this system, and the flow of gases through this system, is as described above. Preferably, the flow of oxygen through the system is controlled in a pulsed manner. However, a continuous supply of oxygen could also be used.

With reference to the third application, an alternative system may be used in combination with a ventilation mask for providing ventilation gas mixtures for use during high altitude sports or activities, such as mountaineering, skydiving, or parachuting for example military parachuting. This system is generally the same as the system described above in connection with the third application. However, in this application, the gas reservoir comprises an exhaust valve configured to provide an increased pressure restriction, facilitating operation of the system at pressures greater than ambient air pressure. By this we mean that the pressure within the gas mixing device is higher than the ambient pressure outside the system and so by configuring the exhaust valve to release a higher pressure, the system is forcing the ventilation gas mixture out of the gas mixing device at into a user's lungs under pressure. In this case, i.e. during high altitude sport or activity applications, oxygen being supplied through the first gas feed is supplied to the gas mixing device at pressures that are greater than those of oxygen being supplied through the first gas feed when the system is being used for other applications. This has the advantage that the partial pressure of oxygen and carbon dioxide can be increased in order to make them more effectively used by the user, whilst still conserving the gases within the system which optimises gas usage. In some applications, this pressure restriction may be controlled based upon the altitude of the system, and/or the gas composition measured within the gas mixing device. This system may also be used in combination with a ventilation mask for providing ventilation gas mixtures for use during medical applications allowing increased partial pressures of gases than would be achieved at a user's ambient pressure

## Claims

1. An apparatus for preparing a ventilation gas mixture for a user comprising:
a ventilation mask (41) configured to receive oxygen through a first gas feed (6), the ventilation mask being further configured to received exhaled air from a person;
a re-breathing bag (11) coupled to the ventilation mask via a second gas feed (12), the re-breathing bag configured to receive the exhaled air from the ventilation mask through the second gas feed (12), wherein the re-breathing bag is further configured to re-supply the exhaled air to the ventilation mask through the second gas feed;
an altitude sensor (14) configured to determine altitude information;
a control system (16) configured to receive the altitude information from the altitude sensor and further configured to control the flow of oxygen through the first gas feed based on the received altitude information;
wherein the ventilation mask is arranged to combine the oxygen received through the first gas feed with the re-supplied exhaled air received through the second gas feed in order to prepare a ventilation gas mixture.

2. The apparatus according to claim 1 wherein the altitude sensor comprises a pressure sensor (15).

3. The apparatus according to claim 2 wherein the pressure sensor arranged to determine the pressure of the ambient air and further configured to send the ambient air pressure to the control system.

4. The apparatus according to claim 2 wherein the pressure sensor arranged to determine the pressure of the exhaled air and further configured to send the exhaled air pressure to the control system.

5. The apparatus according to claim 4 wherein the pressure sensor is located within the first gas feed.

6. The apparatus according to claim 4 wherein the pressure sensor is located within the ventilation mask.

7. The apparatus according to any preceding claim further comprising an oxygen sensor (28) configured to determine the partial pressure of oxygen within the re-breathing bag and send the partial pressure of oxygen to the control system, wherein the control system is configured to control the flow of oxygen through the first gas feed based on the partial pressure of oxygen in the re-breathing bag.

8. The apparatus according to any preceding claim further comprising a carbon dioxide sensor (30) configured to determine the partial pressure of carbon dioxide within the re-breathing bag and send the partial pressure of carbon dioxide to the control system, wherein the control system is configured to control the flow of oxygen through the first gas feed based on the partial pressure of carbon dioxide in the re-breathing bag.

9. The apparatus according to any preceding claim wherein the ventilation mask comprises a third gas feed (32) configured to receive ambient air.

10. The apparatus according to clam 9 wherein the control system is configured to control the flow of ambient air through the third gas feed into the ventilation mask based on the received altitude information.

11. The apparatus according to any preceding claim wherein the control system is configured to control the supply of oxygen through the first gas feed such that oxygen is supplied to the ventilation mask as a at least one pulse, wherein the at least one pulse comprises a predetermined volume of oxygen.

12. The apparatus according to claim 11 wherein each pulse in the plurality of pulses comprises the same volume of oxygen.

13. The apparatus according to claim 11 wherein at least two pulses in the plurality of pulses comprise a different volume of oxygen.

14. The apparatus according to any preceding claim wherein the control system is configured to control the supply of oxygen through the first gas feed such that oxygen is supplied to the ventilation mask as a continuous flow of oxygen.

15. A method of preparing a ventilation gas mixture comprising the steps of:
supplying oxygen to a ventilation mask through a first gas feed;
receiving, by the ventilation mask, exhaled air from a person;
supplying the exhaled air from the ventilation mask to a re-breathing bag through a second gas feed;
re-supplying the exhaled air from the re-breathing bag to the ventilation mask through the second gas feed;
determining the altitude of the ventilation mask using an altitude sensor and sending the altitude to a control system;
controlling, by the control system, the flow of oxygen through the first gas feed based on the received altitude;
combining, by the ventilation mask, the oxygen received through the first gas feed with the re-supplied exhaled air received through the second gas feed in order to prepare the ventilation gas mixture.

## Patentansprüche

1. Vorrichtung zur Herstellung eines Beatmungsgasgemischs für einen Benutzer, umfassend:
eine Beatmungsmaske (41), die so konfiguriert ist, dass sie Sauerstoff durch eine erste Gaszufuhr (6) empfängt, wobei die Beatmungsmaske ferner so konfiguriert ist, dass sie ausgeatmete Luft einer Person empfängt;
einen Rückatembeutel (11), der über eine zweite Gaszufuhr (12) an die Beatmungsmaske gekoppelt ist, wobei der Rückatembeutel so konfiguriert ist, dass er die ausgeatmete Luft aus der Beatmungsmaske durch die zweite Gaszufuhr (12) aufnimmt, wobei der Rückatembeutel ferner so konfiguriert ist, dass er die ausgeatmete Luft durch die zweite Gaszufuhr wieder der Beatmungsmaske zuführt;
einen Höhensensor (14), der so konfiguriert ist, dass er Höheninformationen bestimmt;
ein Steuersystem (16), das so konfiguriert ist, dass es die Höheninformationen von dem Höhensensor empfängt und ferner so konfiguriert ist, dass es den Sauerstofffluss durch die erste Gaszufuhr basierend auf den empfangenen Höheninformationen steuert;
wobei die Beatmungsmaske so angeordnet ist, dass sie den durch die erste Gaszufuhr empfangenen Sauerstoff mit der durch die zweite Gaszufuhr zurückgeführten ausgeatmeten Luft kombiniert, um ein Beatmungsgasgemisch herzustellen.

2. Vorrichtung nach Anspruch 1, wobei der Höhensensor einen Drucksensor (15) umfasst.

3. Vorrichtung nach Anspruch 2, wobei der Drucksensor angeordnet ist, um den Druck der Umgebungsluft zu bestimmen, und ferner so konfiguriert ist, dass er den Umgebungsluftdruck an das Steuersystem sendet.

4. Vorrichtung nach Anspruch 2, wobei der Drucksensor angeordnet ist, um den Druck der ausgeatmeten Luft zu bestimmen, und ferner so konfiguriert ist, dass er den ausgeatmeten Luftdruck an das Steuersystem sendet.

5. Vorrichtung nach Anspruch 4, wobei sich der Drucksensor innerhalb der ersten Gaszufuhr befindet.

6. Vorrichtung nach Anspruch 4, wobei sich der Drucksensor innerhalb der Beatmungsmaske befindet.

7. Vorrichtung nach einem der vorhergehenden Ansprüche, die ferner einen Sauerstoffsensor (28) umfasst, der konfiguriert ist, um den Sauerstoffpartialdruck innerhalb des Rückatembeutels zu bestimmen und den Sauerstoffpartialdruck an das Steuersystem zu senden, wobei das Steuersystem so konfiguriert ist, dass es den Sauerstofffluss durch die erste Gaszufuhr basierend auf dem Sauerstoffpartialdruck in dem Rückatembeutel steuert.

8. Vorrichtung nach einem der vorhergehenden Ansprüche, die ferner ein Kohlendioxidsensor (30) umfasst, der konfiguriert ist, um den Teildruck von Kohlendioxid innerhalb des Rückatembeutels zu bestimmen und den Teildruck von Kohlendioxid an das Steuersystem zu senden, wobei das Steuersystem so konfiguriert ist, dass es den Sauerstofffluss durch die erste Gaszufuhr basierend auf dem Partialdruck von Kohlendioxid im Rückatembeutel steuert.

9. Vorrichtung nach einem der vorstehenden Ansprüche, wobei die Beatmungsmaske eine dritte Gaszufuhr (32) umfasst, die so konfiguriert ist, dass sie Umgebungsluft aufnimmt.

10. Vorrichtung nach Anspruch 9, wobei das Steuersystem so konfiguriert ist, dass es den Umgebungsluftfluss durch die dritte Gaszufuhr in die Beatmungsmaske basierend auf den empfangenen Höheninformationen steuert.

11. Vorrichtung nach einem der vorstehenden Ansprüche, wobei das Steuersystem so konfiguriert ist, dass es die Zufuhr von Sauerstoff durch die erste Gaszufuhr steuert, sodass der Beatmungsmaske mindestens ein Impuls Sauerstoff zugeführt wird, wobei der mindestens eine Impuls ein vorbestimmtes Sauerstoffvolumen umfasst.

12. Vorrichtung nach Anspruch 11, wobei jeder Puls in der Vielzahl von Pulsen das gleiche Sauerstoffvolumen umfasst.

13. Vorrichtung nach Anspruch 11, wobei mindestens zwei Pulse in der Vielzahl von Pulsen ein unterschiedliches Sauerstoffvolumen umfassen.

14. Vorrichtung nach einem der vorstehenden Ansprüche, wobei das Steuersystem so konfiguriert ist, dass es die Zufuhr von Sauerstoff durch die erste Gaszufuhr steuert, sodass die Beatmungsmaske mit einem kontinuierlichen Sauerstofffluss versorgt wird.

15. Verfahren zur Herstellung eines Beatmungsgasgemischs, umfassend folgende Schritte:
Zuführen von Sauerstoff zu einer Beatmungsmaske durch eine erste Gaszufuhr;
Empfangen von ausgeatmeter Luft einer Person durch die Beatmungsmaske;
Zuführen von ausgeatmeter Luft aus der Beatmungsmaske an einen Rückatembeutel durch eine zweite Gaszufuhr;
Wiederzuführen der ausgeatmeten Luft aus dem Rückatembeutel zu der Beatmungsmaske durch die zweite Gaszufuhr;
Bestimmen der Höhe der Beatmungsmaske mithilfe eines Höhensensors und Senden der Höhe an ein Steuersystem;
Steuern des Sauerstoffflusses durch die erste Gaszufuhr durch das Steuersystem basierend auf der empfangenen Höhe;
Kombinieren des durch die Beatmungsmaske durch die erste Gaszufuhr erhaltenen Sauerstoffs mit der durch die zweite Gaszufuhr zurückgeführten ausgeatmeten Luft, um das Beatmungsgasgemisch aufzubereiten.

## Revendications

1. Appareil de préparation d'un mélange de gaz de ventilation pour un utilisateur comprenant :
un masque de ventilation (41) configuré pour recevoir de l'oxygène via une première alimentation (6) en gaz, le masque de ventilation étant en outre configuré pour recevoir de l'air expiré d'une personne ;
un ballon de réinspiration (11) couplé au masque de ventilation via une deuxième alimentation (12) en gaz, le ballon de réinspiration étant configuré pour recevoir l'air expiré du masque de ventilation via la deuxième alimentation (12) en gaz, dans lequel le ballon de réinspiration est en outre configuré pour réapprovisionner l'air expiré dans le masque de ventilation via la deuxième alimentation en gaz ;
un capteur d'altitude (14) configuré pour déterminer des informations d'altitude ;
un système de commande (16) configuré pour recevoir les informations d'altitude du capteur d'altitude et en outre configuré pour commander le débit d'oxygène via la première alimentation en gaz sur la base des informations d'altitude reçues ;
dans lequel le masque de ventilation est agencé pour combiner l'oxygène reçu via la première alimentation en gaz avec l'air expiré réapprovisionné reçu via la deuxième alimentation en gaz afin de préparer un mélange de gaz de ventilation.

2. Appareil selon la revendication 1 dans lequel le capteur d'altitude comprend un capteur de pression (15).

3. Appareil selon la revendication 2 dans lequel le capteur de pression est agencé pour déterminer la pression de l'air ambiant et est en outre configuré pour envoyer la pression d'air ambiant au système de commande.

4. Appareil selon la revendication 2 dans lequel le capteur de pression est agencé pour déterminer la pression de l'air expiré et est en outre configuré pour envoyer la pression d'air expiré au système de commande.

5. Appareil selon la revendication 4 dans lequel le capteur de pression est situé à l'intérieur de la première alimentation en gaz.

6. Appareil selon la revendication 4 dans lequel le capteur de pression est situé à l'intérieur du masque de ventilation.

7. Appareil selon l'une quelconque des revendications précédentes comprenant en outre un capteur (28) d'oxygène configuré pour déterminer la pression partielle d'oxygène à l'intérieur du ballon de réinspiration et envoyer la pression partielle d'oxygène au système de commande, dans lequel le système de commande est configuré pour commander le débit d'oxygène via la première alimentation en gaz sur la base de la pression partielle d'oxygène dans le ballon de réinspiration.

8. Appareil selon l'une quelconque des revendications précédentes comprenant en outre un capteur (30) de dioxyde de carbone configuré pour déterminer la pression partielle de dioxyde de carbone à l'intérieur du ballon de réinspiration et envoyer la pression partielle de dioxyde de carbone au système de commande, dans lequel le système de commande est configuré pour commander le débit d'oxygène via la première alimentation en gaz sur la base de la pression partielle de dioxyde de carbone dans le ballon de réinspiration.

9. Appareil selon l'une quelconque des revendications précédentes dans lequel le masque de ventilation comprend une troisième alimentation (32) en gaz configurée pour recevoir de l'air ambiant.

10. Appareil selon la revendication 9 dans lequel le système de commande est configuré pour commander le débit d'air ambiant via la troisième alimentation en gaz dans le masque de ventilation sur la base des informations d'altitude reçues.

11. Appareil selon l'une quelconque des revendications précédentes dans lequel le système de commande est configuré pour commander l'approvisionnement en oxygène via la première alimentation en gaz de telle sorte que l'oxygène soit approvisionné dans le masque de ventilation sous la forme d'au moins une impulsion, dans lequel l'au moins une impulsion comprend un volume prédéterminé d'oxygène.

12. Appareil selon la revendication 11 dans lequel chaque impulsion dans la pluralité d'impulsions comprend le même volume d'oxygène.

13. Appareil selon la revendication 11 dans lequel au moins deux impulsions dans la pluralité d'impulsions comprennent un volume différent d'oxygène.

14. Appareil selon l'une quelconque des revendications précédentes dans lequel le système de commande est configuré pour commander l'approvisionnement en oxygène via la première alimentation en gaz de sorte que l'oxygène soit approvisionné dans le masque de ventilation sous forme de flux continu d'oxygène.

15. Procédé de préparation d'un mélange de gaz de ventilation comprenant les étapes suivantes :
l'approvisionnement en oxygène d'un masque de ventilation via une première alimentation en gaz ;
la réception, par le masque de ventilation, de l'air expiré d'une personne ;
l'approvisionnement de l'air expiré du masque de ventilation dans un ballon de réinspiration via une deuxième alimentation en gaz ;
le réapprovisionnement de l'air expiré du ballon de réinspiration dans le masque de ventilation via la deuxième alimentation en gaz ;
la détermination de l'altitude du masque de ventilation à l'aide d'un capteur d'altitude et l'envoi de l'altitude à un système de commande ;
la commande, par le système de commande, du débit d'oxygène via la première alimentation en gaz sur la base de l'altitude reçue ;
la combinaison, par le masque de ventilation, de l'oxygène reçu via la première alimentation en gaz avec l'air expiré réapprovisionné reçu via la deuxième alimentation en gaz afin de préparer le mélange de gaz de ventilation.
